Europäisches Patentamt

European Patent Office

Office européen des brevets

(19)

(11) Veröffentlichungsnummer: **0 173 185 B1**

(12) **EUROPÄISCHE PATENTSCHRIFT**

(45) Veröffentlichungstag der Patentschrift:
**31.01.90**

(21) Anmeldenummer: **85110273.1**

(22) Anmeldetag: **16.08.85**

(51) Int. Cl.⁴: **C 07 D 233/32**, C 07 D 491/048, C 07 D 495/04, C 07 B 57/00 // (C07D491/048, 235:00, 307:00),(C07D495/04, 235:00, 333:00)

(54) Verfahren zur Herstellung von (3a5,6aR)-und/oder(3aR,6a5)-1,3-Dibenzyi-hexahydro-1H-furo(3,4-d)imidazol-2,4-dion.

(30) Priorität: **25.08.84 DE 3431294**
**21.06.85 DE 3522145**

(43) Veröffentlichungstag der Anmeldung:
**05.03.86 Patentblatt 86/10**

(45) Bekanntmachung des Hinweises auf die Patenterteilung:
**31.01.90 Patentblatt 90/5**

(84) Benannte Vertragsstaaten:
**CH DE FR GB IT LI**

(56) Entgegenhaltungen:
**EP-A- 0 081 047**
**EP-A- 0 082 396**
**EP-A- 0 084 892**
**EP-A- 0 092 194**
**DE-C- 2 058 234**
**DE-C- 2 331 244**

(73) Patentinhaber: **MERCK PATENT GESELLSCHAFT MIT BESCHRÄNKTER HAFTUNG, Frankfurter Strasse 250 Postfach 4119, D-6100 Darmstadt (DE)**

(72) Erfinder: **Reiff, Fritz, Dr., Im Rassdorf 6, D-6104 Seeheim (DE)**
Erfinder: **Müller, Hans-Rudolf, Dr., Am Beckenwäldil 18, CH-8207 Schaffhausen (CH)**
Erfinder: **Hedinger, Alfred, Dorfstrasse 9, CH-8240 Thayngen (CH)**
Erfinder: **Herold, Thomas, Dr., Bergweg 5, D-6101 Brensbach-Wallbach (DE)**
Erfinder: **Casutt, Michael, Dr., Adolf-Kolbing-Strasse 19, D-6102 Pfungstadt (DE)**
Erfinder: **Schweickert, Norbert, Dr., Am Gaishof 11, D-7801 Ebringen (DE)**
Erfinder: **Stöhr, Josef-Stephan, Mozartstrasse 21, D-6085 Nauheim (DE)**
Erfinder: **Kuhn, Walter, Dr., Weidenweg 8, D-8750 Aschaffenburg (DE)**

ACTORUM AG

**Beschreibung**

Die Erfindung betrifft ein neues Verfahren zur Herstellung von
(3aS, 6aR)- und/oder (3aR,6aS)-1,3-Dibenzyl-hexahydro-1H-furo[3,4-d]-imidazol-2,4-dion aus
1,3-Dibenzyl-5-alkoxycarbonyl-2-oxoimidazolidin-4-carbonsäuren,
das darin besteht, daß man ein diastereomeres Salz aus einem optisch aktiven Amin und einer optisch aktiven
1,3-Dibenzyl-5-alkoxycarbonyl-2-oxoimidazolidin-4-carbonsäure
mit komplexen Hydriden reduziert.

(4S, 5R)-1,3-Dibenzyl-5-alkyloxycarbonyl-2-oxo-imidazolidin-4-carbonsäuren
sind wertvolle Zwischenprodukte für die Herstellung von
(3aS, 6aR)-1,3-Dibenzyl-tetrahydro-4H-furo[3,4-d]-imidazol-2,4(1H)-dion,
einem Schlüsselprodukt für die Herstellung von (+)-Biotin.

Von den bisher bekannten Verfahren zur optischen Spaltung von racemischen Halbestern der Formel Ia/b beschreibt DBP 2 058 234 die Herstellung von Cyclohexylhalbestern und die Trennung der daraus erhaltenen diastereomeren (+)-Ephedriniumsalze durch fraktionierte Kristallisation sowie die Herstellung von diastereomeren Cholesterinhalbestern und die Trennung der daraus erhaltenen Triethylammoniumsalze durch fraktionierte Kristallisation. Die erzielten Ausbeuten bei diesen Verfahren liegen jedoch unter 50% an theoretisch erzielbaren reinen Isomeren. Das chirale Hilfsreagenz Cholesterin besitzt weiterhin den Nachteil eines hohen Preises und ist auch nur unvollständig wiedergewinnbar.

In EP-OS 0 092 194 wird die optische Spaltung der racemischen Methyl- und Ethylhalbester durch Bildung der diastereomeren Salzpaare mit einem optisch aktiven 1,2-Diphenylethanamin und deren Trennung durch Kristallisation beschrieben. Als weitere Methode wird die Spontankristallisation eines Enantiomeren aus der übersättigten Lösung des Racemates durch Animpfen mit dem gewünschten Enantiomeren angegeben. Auch diese Verfahren leiden unter der geringen Trennausbeute, die nur etwa 30% d.Th. beträgt. Es bestand daher die Aufgabe, ein Verfahren zur optischen Spaltung von Halbestern der Formel Ia/b zu finden, das deren Trennung in hoher Ausbeute unter Verwendung eines wohlfeilen Spaltungsreagenzes erlaubt.

Es wurde jetzt überraschend gefunden, daß Racemate der Formel Ia/b mittels (+)-Dehydroabietylamin in hohen Ausbeuten und großer Selektivität in die Enantiomeren gespalten werden können.

Gegenstand der Erfindung sind die neuen (+)-Dehydroabietylaminsalze von (4S, 5R)- und (4R, 5S)-cis-1,3-Dibenzyl-5-alkyloxycarbonyl-2-oxo-imidazolidin-4-carbonsäuren, worin -alkyl- Alkyl mit 1 bis 6 C-Atomen, Alkoxyalkyl mit 2 bis 8 C-Atomen, Cycloalkyl mit 3 bis 5 C-Atomen, Alkenyl mit 2 bis 6 C-Atomen, Benzyl oder 1- oder 2-Phenylethyl bedeutet.

Die Trennung racemischer Halbester erfolgt nach an sich bekannten Standardverfahren zur optischen Spaltung von Racematen (z.B. P.H. Boyle, Quart. Rev. 25 (1971) 323–341). Racemate lassen sich durch Chromatographie an chiralen Phasen, durch Impfung übersättigter Lösungen oder durch mechanische Auslese trennen. Eine weitere Trennmethode besteht in der Bildung, Trennung oder Zersetzung von Diastereomeren. Ein bevorzugtes Verfahren ist die Trennung diastereomerer Salzpaare. Ein für die Spaltung der Racemate der Formel Ia/b besonders bevorzugtes Verfahren besteht im Lösen der Racemate zusammen mit einer bestimmten Menge (+)-Dehydroabietylamin in einem geeigneten organischen Lösungsmittel und darauffolgender Kristallisation des (+)-Dehydroabietylaminsalzes eines Enantiomeren. Nach dessen Abtrennung durch Filtration oder Zentrifugieren läßt sich aus der verbleibenden Lösung nach Entfernen des Lösungsmittels und gegebenenfalls Kristallisation des Rückstandes das andere Enantiomere erhalten.

Die Menge an angewandtem (+)-Dehydroabietylamin beträgt 0,5 bis 1 Mol, bevorzugt 0,5 bis 0,7 Mol, bezogen auf 1 Mol zu spaltendes Racemat. Als Lösungsmittel für die Trennung eignen sich Wasser oder inerte organische Lösungsmittel, vorzugsweise wassermischbare Lösungsmittel, insbesondere Alkohole wie Methanol, Ethanol, Isopropanol, n-Butanol oder tert. Butanol; Ether wie Tetrahydrofuran, Dioxan, Ethylenglykolmonomethyl- oder -monoethylether (Methylglykol oder Ethylglykol), Ethylenglykoldimethylether (Diglyme); Ketone wie Acetone, Butanon oder Isobutylmethylketon; Nitrile wie Acetonitril; Nitroverbindungen wie Nitromethan.

Bevorzugte Lösungsmittel sind Alkohole, Ketone und Ether, besonders bevorzugt Methanol, Ethanol, Isopropanol, Aceton oder Tetrahydrofuran.

Weiter eignen sich Gemische dieser Lösungsmittel untereinander. Bevorzugt sind Mischungen dieser Lösungsmittel mit Wasser in Anteilen von 0,5 bis 50%, insbesondere von 0,5 bis 10% und besonders bevorzugt von 1 bis 5%.

Die bei der Durchführung dieses Verfahrens angewandten Temperaturen liegen zweckmäßig zwischen −20° und dem Siedepunkt des verwendeten Lösungsmittels, vorzugsweise jedoch unter der Kristallisationstemperatur des zu kristallisierenden Enantiomerensalzpaares.

Für die optische Spaltung nach diesem Verfahren besonders bevorzugt sind die Verbindungen der Formel Ia/b, in denen R Methyl oder Ethyl bedeutet.

Eine weitere bevorzugte Ausführungsform dieses Verfahrens beteht darin, daß man das diastereomere Salzpaargemisch fraktioniert kristallisiert. Hierzu wird das Racemat der Halbester der Formel Ia/b zunächst mit der äquivalenten Menge (+)-Dehydroabietylamin vollständig in das Gemisch der diastereomeren Salzpaare überführt. Die Umsetzung der racemischen Halbester mit dem optisch aktiven Amin erfolgt ohne Lösungsmittel oder gelöst in einem organischen Lösungsmittel, vorzugsweise in einem unpolaren Lösungsmittel, insbesondere chlorierten Kohlenwasserstoffen wie Methylenchlorid, Chloroform, Trichlorethylen, 1,2-Dichlorethan oder Kohlenstofftetrachlorid oder Kohlenwasserstoffen wie Hexan, Petrolether, Benzol, Toluol oder Xylol. Weiterhin eignen sich auch Gemische dieser Lösungsmittel untereinander.

Die nach Entfernen des Lösungsmittels isolierten Salze werden zur optischen Spaltung in den zuvor zur Trennung der racemischen Halbester beschriebenen Lösungsmitteln kristallisiert. Eine bevorzugte Ausführungsform dieses Verfahrens besteht in der optischen Spaltung der (+)-Dehydroabietylaminsalze von cis-1,3-Dibenzyl-5-methoxycarbonyl-2-oxo-imidazolidin-4-carbonsäure und cis-1,3-Dibenzyl-5-ethoxycarbonyl-2-oxo-imidazolidin-4-carbonsäure.

Die nach diesem Verfahren erhaltenen (+)-Dehydroabietylaminsalze der optisch aktiven Halbester la oder lb lassen sich durch Behandeln mit einer Säure oder Base in die freien Halbestersäuren zurückführen. Geeignete Basen sind z.B. Alkali- und Erdalkalihydroxide, -carbonate und -hydrogencarbonate sowie Ammoniak und starke organische Basen. Bevorzugte Basen sind Alkalihydroxide und Ammoniak, insbesondere Kalium- und Natriumhydroxid.

Als Säuren geeignet sind starke organische Säuren, z.B. Ameisensäure, Essigsäure, ferner anorganische Säuren, z.B. Phosphorsäuren, Salpetersäure, vorzugsweise Mineralsäuren wie Chlorwasserstoffsäure oder Bromwasserstoffsäure. Besonders bevorzugt ist Schwefelsäure, mit der (+)-Dehydroabietylamin ein schwerlösliches, gut kristallisierendes und leicht filtrierbares Hydrogensulfat bildet, das die Rückgewinnung des Spaltungsmittels in hoher Ausbeute erlaubt.

Allgemein bevorzugt zur Freisetzung der optisch aktiven Halbester der Formel la/b aus ihren Dehydroabietylaminsalzen sind Säuren, da die Halbester gegenüber Basen eine verminderte Stabilität aufweisen.

Die Menge an verwendeter Säure ist in weiten Grenzen variierbar, mindestens wird jedoch 1 Mol, vorzugsweise 1 bis 1,5 Mol auf 1 Mol zu zerlegendes Diastereomerensalzpaar verwendet.

Die Freisetzung des Halbesters erfolgt durch Zugabe der Säure zu einer Lösung des Diastereomerenpaares in einem geeigneten Lösungsmittel, vorzugsweise Wasser. Mittels eines organischen Lösungsmittels wie z.B. Diethylether, Toluol, Essigester, Dichlormethan oder auch deren Gemischen läßt sich dann der freigesetzte Halbester extrahieren. Gegebenenfalls nach Waschen des Extraktes wird das Lösungsmittel entfernt und so der gewünchte optisch aktive Halbester erhalten.

Zur Wiedergewinnung des als Spaltungsreagenz eingesetzten (+)-Dehydroabietylamins kann dieses nach Alkalisieren der extrahierten Lösung durch Zusatz einer starken Base wie z.B. Ammoniak, Natron- oder Kalilauge in Freiheit gesetzt werden und anschließend analog zu der für die Isolierung des optisch aktiven Halbesters beschriebenen Weise extrahiert werden. Bei Verwendung der als besonders bevorzugt bezeichneten Schwefelsäure zur Zerlegung des Diastereomerensalzpaares fällt das (+)-Dehydroabietylamin in Form des schwer löslichen Hydrogensulfates an. Nach dessen Abtrennung durch Filtration oder Zentrifugieren läßt sich die freie Base durch Versetzen mit starken Laugen und anschließender Extraktion auf die vorstehende Art und Weise gewinnen.

Die Herstellung der als Ausgangsmaterial benötigten racemischen Halbester der Formel la/b aus z.B. cis-1,3-Dibenzyl-2-oxo-imidazolidin-4,5-dicarbonsäureanhydrid ist z.B. aus DBP 2 058 234 oder EP-OS 0 092 194 bekannt und erfolgt durch Umsetzung mit dem entsprechenden Alkohol in einem inerten organischen Lösungsmittel, wie z.B. Benzol, Toluol oder Xylol, zweckmäßigerweise bei erhöhter Temperatur.

Die so erhaltenen optisch aktiven cis-1,3-Dibenzyl-5-alkyloxycarbonyl-2-oxo-imidazolidin-4-carbonsäuren lassen sich wie z.B. beschrieben bei Gerecke et al., Helv. Chim. Acta 53 (1970) 991–999, zum optisch aktiven 1,3-Dibenzyl-tetra-hydro-4H-furo[3,4-d]imidazol-2,4(1H)-dion reduzieren. Dabei kann vom freien optisch aktiven Halbester ausgegangen werden. Es ist jedoch gleichermaßen möglich, dessen diastereomeres (+)-Dehydroabietylaminsalz als Ausgangsmaterial für die Reduktion einzusetzen. Ein solches Verfahren bietet besondere Vorteile.

Aus der DE-PS 2 058 248 ist bereits ein Verfahren zur Herstellung von (3aS, 6aR)-Lacton bekannt, wobei cis-1,3-Dibenzyl-2-oxo-imidazolidin-4,5-dicarbonsäure-cholesteryl- oder -cyclohexylhalbester in ihre Enantiomere getrennt werden und das entsprechende Enantiomere durch Reduktion in das gewünschte Lacton überführt wird. Die erzielten Ausbeuten bei diesem Verfahren liegen jedoch unter 50% an theoretisch erzielbaren reinen Isomeren. Das chirale Hilfsreagenz Cholesterin ist vergleichsweise teuer und nur unvollständig wiedergewinnbar. Weiterhin muß nach dem beanspruchten Verfahren der unerwünschte enantiomere Halbester wieder recyclisiert werden.

Eine Verbesserung dieses Verfahrens ist aus der EP-OS 0 081 047 bekannt. Hierbei wird die Carboxylgruppe des unerwünschten Halbesters in das Säurechlorid überführt. Nachfolgende Reduktion liefert das richtige (3R, 6R)-Lacton. Die geringe Trennausbeute bei der Diastereomerentrennung und der hohe Preis der chiralen Hilfsstoffe verleiht diesem Verfahren keine wirtschaftliche Bedeutung.

In der DE-PS 2 331 244 wird ein Verfahren zur Herstellung der (3R, 6S)- und (3S, 6R)-Lactone beansprucht, bei dem aus cis-1,3-Dibenzyl-2-oxo-imidazolidin-4,5-dicarbonsäure und einem optisch aktiven Amin erhaltenes cis-1,3-Dibenzyl-hexahydropyrrolo-[3,4-d]imidazol-2,4,6-trion einer asymmetrischen Reduktion unterworfen und der erhaltene Amidalkohol zum gewünschten Lacton hydrolysiert wird.

Auch bei dieser Verfahrensweise sind der hohe Preis des optisch aktiven Amins und die ungenügende Trennausbeute von Nachteil.

Die nach der EP-OS 84 892 in höheren Ausbeuten verlaufende Diastereomerentrennung erfordert ebenfalls ein schwer zugängliches optisch aktives Amin.

In der Salzbildung der enantiomeren cis-1,3-Dibenzyl-2-oxoimidazolidin-4,5-dicarbonsäuren mit (+)-Dehydroabietylamin steht hingegen ein vorteilhaftes Verfahren zur Herstellung der optisch aktiven Halbester zur Verfügung, von denen jeder, wie z.B. in der EP-OS 84 892 beschrieben, in das gewünschte optisch aktive Lacton überführt werden kann.

In den voranstehenden Verfahren werden die Halbester vor der Reduktion aus ihren mit optisch aktiven Aminen gebildeten diastereomeren Salzpaaren durch Behandeln mit einer starken Säure freigesetzt und isoliert.

Im nachfolgenden, separat durchgeführten Reduktionsschritt wird zunächst ein Äquivalent Reduktionsmittel für die Umsetzung mit dem aciden Wasserstoff der Carboxylgruppe des freien Halbesters verbraucht und geht damit der Reduktion verloren.

Es bestand daher die Aufgabe, ein Verfahren zur Herstellung von optisch aktiven 1,3-Dibenzyl-tetrahydro-4H-furo[3,4-d]-imidazol-2,4-dionen durch Reduktion entsprechender cis-1,3-Dibenzyl-2-oxo-imidazolidin-4,5-dicarbonsäurehalbester zu finden, das eine bessere Ausnutzung der Reduktionsmittel bei vereinfachter Arbeitsweise und die Gewinnung von Produkten höherer chemischer und optischer Reinheit gestattet.

Es wurde jetzt überraschend gefunden, daß sich diastereomere Salze aus einem optisch aktiven Amin und einer optisch aktiven 1,3-Dibenzyl-5-alkoxycarbonyl-2-oxoimidazolidin-4-carbonsäure in hohen chemischen und optischen Ausbeuten ohne störenden Einfluß der Aminkomponente reduzieren lassen.

Ein weiterer Gegenstand der Erfindung ist somit ein Verfahren zur Herstellung von (3aS, 6aR)- und/oder (3aR, 6aS)-1,3-Dibenzyl-hexahydro-1H-furo-[3,4-d]imidazol-2,4-dion aus 1,3-Dibenzyl-5-alkoxycarbonyl-2-oxoimidazolidin-4-carbonsäuren, dadurch gekennzeichnet, daß man ein diastereomeres Salz aus einem optisch aktiven Amin und einer optisch aktiven 1,3-Dibenzyl-5-alkoxycarbonyl-2-oxoimidazolidin-4-carbonsäure mit komplexen Hydriden reduziert.

Weiterhin ist Gegenstand der Erfindung die Verwendung eines nach dem erfindungsgemäßen Verfahren erhaltenen optisch aktiven 1,3-Dibenzyl-hexahydro-1H-furo[3,4-d]-imidazol-2,4-dions zur Herstellung von D-(+)-Biotin.

Die erfindungsgemäße Reduktion der diastereomeren Salze der optisch aktiven Halbester erfolgt nach den an sich bekannten Verfahren zur Reduktion der freien Halbester.

Je nach Wahl des Reduktionsmittels können aus Salzen mit 4S, 5R- und 4R, 5S-Konfiguration des Halbesters jeweils sowohl das (3aR, 6aS)- als auch das (3aS, 6aR)-Lacton erhalten werden.

Wird beispielsweise ein Salz eines 4S, 5R-Halbesters an der Carboxylgruppe reduziert, erhält man das 3aR, 6aS-Lacton, reduziert man die Alkoxycarbonylgruppe, wird ein 3aS, 6aR-Lacton erhalten. Für die 4R, 5S-Halbester gelten die umgekehrten Zusammenhänge.

Zur Reduktion der Säuregruppe geeignete Reduktionsmittel sind beispielsweise Borhydride, wie Diboran.

Als Lösungsmittel eignen sich gegenüber dem Reduktionsmittel inerte organische Lösungsmittel, wie beispielsweise Kohlenwasserstoffe wie Benzol, Toluol, und Ether wie Diethylether, Ethylenglykol- und Diethylenglycol-dialkylether, Tetrahydrofuran, Dioxan. Besonders bevorzugt sind cyclische Ether wie Tetrahydrofuran und Dioxan. Es können auch Gemische dieser Lösungsmittel verwendet werden.

Die Temperatur während der Reduktion beträgt zweckmäßig zwischen $-20$ und $+40°$, vorzugsweise von $-10$ bis $+30°$, insbesondere zwischen $0$ und $+20°$. Die Menge an verwendetem Hydrid liegt zwischen 0,8 und 3 Äquivalenten der theoretisch erforderlichen Menge, vorzugsweise zwischen 1 und 2 Äquivalenten und insbesondere zwischen 1 und 1,5 Äquivalenten. So liegt die bevorzugte Menge bei Verwendung von Diboran bei 0,5 bis 0,75 Mol $B_2H_6$ je Mol Halbestersalz.

Diboran kann als solches zur Reduktion eingesetzt werden, es kann aber auch in situ, beispielsweise aus $NaBH_4$ und einer Lewis-Säure wie Bortrifluorid oder dessen Diethyletheraddukt nach bekannten Methoden hergestellt werden, wobei dies vorzugsweise in für die Reduktion der Halbestersalze geeigneten Lösungsmitteln erfolgt, so daß die erhaltene Lösung von Diboran auch als Reaktionsmedium für die nachfolgende Reduktion dienen kann.

Zur Reduktion der Alkoxycarbonylgruppe geeignete Reduktionsmittel sind beispielsweise komplexe Borhydride, wie Lithiumborhydrid, Natriumborhydrid, Calciumborhydrid oder Aluminiumhydride wie Diisobutylaluminiumhydrid und Diethylaluminiumnatriumhydrid.

Die Reduktion erfolgt zweckmäßig in einem gegenüber den Reduktionsmitteln unter den Reaktionsbedingungen inerten Lösungsmittel, in welchem die Reaktionspartner zumindest teilweise löslich sind.

Geeignete Lösungsmittel für in wässrigem Medium einsetzbare Reduktionsmittel wie Natriumborhydrid eignen sich beispielsweise Wasser, Alkohole wie Methanol, Ethanol, Isopropanol, Ethylenglykol, Diethylenglykol, Ether wie Tetrahydrofuran, Dioxan. Es können auch Gemische dieser Lösungsmittel untereinander oder mit nichtwassermischbaren Lösungsmitteln verwendet werden.

Als Lösungsmittel für mit Wasser reagierende Reduktionsmittel eignen sich neben Ethern wie Diethylether, Ethylenglykol- und Diethylenglykoldialkylethern, Tetrahydrofuran, Dioxan insbesondere Kohlenwasserstoffe wie Benzol und Toluol. Auch Gemische dieser Lösungsmittel können angewendet werden.

Die Reaktionstemperaturen liegen zwischen −70 und +100°. Aluminiumhaltige Reduktionsmittel werden vorzugsweise bei −70 bis +30°, insbesondere bei −40 bis +30° angewendet. Borhaltige Reduktionsmittel werden vorzugsweise zwischen −10 und 100°, insbesondere zwischen 0° und 80° angewandt.

Die Menge an angewandtem Reduktionsmittel liegt zwischen 0,8 und 3 Äquivalenten der theoretisch erforderlichen Menge, insbesondere zwischen 1 und 2 Äquivalenten und vorzugsweise zwischen 1 und 1,5 Äquivalenten. So liegt die bevorzugte Menge bei Verwendung von Natriumborhydrid bei 0,75 bis 1,125 Mol $NaBH_4$ je Mol Halbestersalz.

In den als Ausgangsstoffen verwendeten diastereomeren Salzen von 1,3-Dibenzyl-5-alkoxycarbonyl-2-oxoimidazolidin-4-carbonsäuren bedeutet -alkoxycarbonyl- -alkyloxycarbonyl- mit 1 bis 6 C-Atomen, -alkoxyalkoxycarbonyl- mit 2 bis 8 C-Atomen, -alkenyloxycarbonyl- mit 2 bis 6 C-Atomen, -benzyloxycarbonyl-, oder 1- oder 2-phenylethoxycarbonyl-.

Für die Reduktion nach dem erfindungsgemäßen Verfahren besonders bevorzugt sind die Salze der 1,3-Dibenzyl-5-methoxy-, -5-ethoxy- und -5-benzyloxycarbonyl-2-oxo-imidazolidin-4-carbonsäuren mit optisch aktiven Aminen.

Geeignete optisch aktive Aminsalzkomponenten sind alle zur Trennung der racemischen Halbester geeigneten optisch aktiven Amine wie beispielsweise das in der DE-PS 2 058 248 beanspruchte Ephedrin, die in der EP-OS 92 194 genannten 1,2-Diphenylethanamine und insbesondere (+)-Dehydroabietylamin. Die Verwendung diastereomerer (+)-Dehydroabietylaminsalze stellt eine bevorzugte Ausführungsform des erfindungsgemäßen Verfahrens dar.

Die Durchführung des Verfahrens gestaltet sich einfach. Die als Ausgangsmaterial einzusetzenden diastereomeren Salze sind bekannt oder können nach an sich bekannten Methoden wie sie z.B. in der EP-OS 92 194 angegeben sind, hergestellt werden. Die Ausführung der Reaktion erfolgt durch Vermischen der Komponenten sowie Erwärmen oder Kühlen des gerührten Reaktionsgemisches auf die zur Umsetzung erforderliche Temperatur, wobei es jedoch von Vorteil sein kann, das diastereomere Salz, gegebenenfalls gelöst in einem geeigneten Lösungsmittel, langsam zu dem bereits vorgelegten Reduktionsmittel zuzugeben. Die Reaktionszeiten betragen 0,5 bis 40 Stunden, vorzugsweise 1 bis 8 Stunden.

Nach Durchführung der Reaktion wird das Reaktionsgemisch durch Zusatz einer Säure, beispielsweise einer Mineralsäure wie Salzsäure oder Schwefelsäure, sauer gestellt und das Reduktionsprodukt in Form des optisch aktiven Lactons mit einem geeigneten Lösungsmittel wie z.B. Diethylether, Ethylacetat, Dichlormethan, Chloroform oder Toluol extrahiert.

Nach Entfernen des zur Extraktion verwendeten Lösungsmittels hinterbleibt das gewünschte Lacton meist kristallin in hoher Reinheit. Erforderlichenfalls kann es durch Chromatographie oder Kristallisation weiter gereinigt werden.

Zur Wiedergewinnung des optisch aktiven Amins kann dieses nach Alkalisieren der extrahierten Lösung durch Zusatz einer starken Base wie z.B. Ammoniak, Natron- oder Kalilauge in Freiheit gesetzt werden und anschließend analog zu der für die Isolierung des optisch aktiven Lactons beschriebenen Weise extrahiert werden.

Aus optisch aktivem (3aS, 6aR)-1,3-Dibenzyl-tetrahydro-4H-furo[3,4-d]imidazol-2,4(1H)-dion läßt sich in bekannter Weise, wie z.B. in DBP 2 058 234 oder DBP 2 331 244 beschrieben, D-(+)-Biotin erhalten.

In der vorliegenden Erfindung steht daher ein sehr vorteilhaftes Verfahren zur einfachen, ökonomischen und in hoher chemischer und optischer Reinheit verlaufenden Reduktion diastereomerer Salze aus einem optisch aktiven Amin und einer optisch aktiven 1,3-Dibenzyl-5-alkoxycarbonyl-2-oxoimidazolidin-4-carbonsäure zu optisch aktiven 1,3-Dibenzyl-hexahydro-1H-furo[3,4-d]imidazol-2,4-dionen und damit zur Herstellung von D-(+)-Biotin zur Verfügung.

Beispiel 1:
a) Die Suspension von 250 g (0,743 Mol) cis-1,3-Dibenzylhexahydro-1H-furo[3,4-d]-imidazol-2,4,6-trion in 59,7 g (1,283 Mol) Ethanol und 2 Liter Benzol wird 2 Stunden zum Rückfluß erhitzt. Anschließend engt man ein und läßt Kristallisieren.
Ausbeute: 256 g (90% d.Th.) cis-1,3-Dibenzyl-2-oxo-imidazolidin-5-ethoxycarbonyl-4-carbonsäure; F 93°.
b) 57,4 g (0,15 Mol) racemische cis-1,3-Dibenzyl-5-ethoxycarbonyl-2-oxo-imidazolidin-4-carbonsäure und 42,8 g (0,15 Mol) (+)-Dehydroabietylamin werden in 375 ml Tetrahydrofuran gelöst und bei Raumtemperatur mit 7,5 ml Wasser versetzt. Bei −7° kristallisieren 47,7 g (95% d.Th.) (4R, 5S)-cis-1,3-Dibenzyl-5-ethoxycarbonyl-2-oxo-imidazolidin-4-carbonsäure-dehydroabietyl-aminsalz;
$[\alpha]_{365}^{25} = +47,1°$. Nach Zusatz von 400 ml Wasser oder 500 ml Hexan zur Mutterlauge kristallisieren 49,8 g (98% d.Th.) (4S, 5R)-cis-1,3-Dibenzyl-5-ethoxycarbonyl-2-oxo-imidazolidin-4-carbonsäure-dehydroabietyl-aminsalz; $[\alpha]_{365}^{25} = +61,24°$.

Beispiel 2:
Äquivalente Mengen racemischer cis-1,3-Dibenzyl-5-ethoxycarbonyl-2-oxo-imidazolidin-4-carbonsäure und (+)-Dehydroabietylamin werden in Ethanol gelöst (4 bis 5 Ltr. je Mol), mit 10% Wasser versetzt und bei −10° kristallisieren gelassen. Die Ausbeute an (4R, 5S)-cis-1,3-Dibenzyl-5-ethoxycarbonyl-2-oxo-imidazolidin-4-carbonsäure-dehydroabietylaminsalz beträgt 89% d.Th.; $[\alpha]_{365}^{25} = +48,1°$.

Die Mutterlauge wird auf ca. ⅔ ihres Volumens eingeengt und mit der gleichen Menge Wasser verdünnt, worauf sich in 92% d.Th.
(4S, 5R)-cis-1,3-Dibenzyl-5-ethoxycarbonyl-2-oxo-imidazolidin-4-carbonsäure-dehydroabietyl-aminsalz

isolieren läßt; $[\alpha]_{365}^{25} = +63,6°$. Aus der zweiten Mutterlauge wird durch Eindampfen und Behandeln des Rückstandes mit 100 ml Diisopropylether in 9% d.Th. ein Konglomerat der diasteromeren (+)-Dehydroabietylaminsalze der
(4R, 5S)- und (4S, 5R)-cis-1,3-Dibenzyl-5-ethoxycarbonyl-2-oxo-imidazolidin-4-carbonsäuren erhalten, das erneut zur Trennung eingesetzt werden kann.

Beispiel 3:
Die Lösung von 26,8 g (0,07 Mol) racemischer
cis-1,3-Dibenzyl-5-ethoxycarbonyl-2-oxo-4-carbonsäure

und 20 g (0,07 Mol) (+)-Dehydroabietylamin in 80 ml Methylenchlorid wird zum Rückstand eingedampft. Kristallisation aus Diisopropylether liefert 45,6 g (97,5% d.Th.) des diastereomeren Ethyl-halbestersäure- Dehydroabietylamin-Salzpaares; $[\alpha]_{365}^{25} = +54,9°$.

Beispiel 4:
Die Trennung des in Beispiel 3 erhaltenen diastereomeren
(+)-Dehydroabietylamin-cis-1,3-dibenzyl-5-ethoxycarbonyl-2-oxo-imidazolidin-4-carbonsäure-Salzgemisches

gelingt durch Kristallisation aus 10% Wasser enthaltendem Ethanol.
Zunächst kristallisiert das Salz mit der (4R, 5S)-Konfiguration; $[\alpha]_{365}^{25}$ +46,2°.
Aus der Mutterlauge der 1. Kristallisation wird durch Zusatz von Wasser (ca. 50% bezogen auf das Volumen des organischen Lösungsmittels), Diisopropylether oder Petrolether das Salz mit der (4S, 5R)-Konfiguration zur Kristallisation gebracht; $[\alpha]_{365}^{25} = +63,6°$.

Beispiel 5:
Es wird analog zu Beispiel 4 verfahren, wobei jedoch statt Ethanol 5% Wasser enthaltendes Isopropanol verwendet wird. Die Menge an (4R, 5S)-Diastereomerensalzpaar entspricht der in Beispiel 4 erhaltenen.

Beispiel 6:
Es wird analog zu Beispiel 4 verfahren, wobei jedoch statt Ethanol 2% Wasser enthaltendes Tetrahydrofuran verwendet wird. Die Ausbeute entspricht der in Beispiel 4 erhaltenen.

Beispiel 7:
Die Trennung der racemischen
cis-1,3-Dibenzyl-5-methoxycarbonyl-2-oxo-imid-azolidin-4-carbonsäuren

erfolgt analog zu Beispiel 4, wobei jedoch der Wasserzusatz auf 5% verringert wird. Die Ausbeuten entsprechen der bei der Trennung der entsprechenden Ethylhalbester in den Beispielen 4 bis 7 erhaltenen Ausbeuten.
(4S, 5R)-cis-1,3-Dibenzyl-5-methoxycarbonyl-2-oxo-imidazolidin-4-carbonsäure: $[\alpha]_{365}^{25} = +39°$.
(4R, 5S)-cis-1,3-Dibenzyl-5-methoxycarbonyl-2-oxo-imidazolidin-4-carbonsäure: $[\alpha]_{365}^{25} = +67°$.

Beispiel 8:
Es wird analog zu Beispiel 7 verfahren, wobei jedoch statt Ethanol 3% Wasser enthaltendes Iso-propanol verwendet wird. Die Ausbeute ist mit der in Beispiel 7 erreichten vergleichbar.

Beispiel 9:
Es wird analog zu Beispiel 7 verfahren, wobei jedoch statt Ethanol 1% Wasser enthaltendes Tetrahydrofuran verwendet wird. Die Ausbeute liegt mit der in Beispiel 7 erzielten auf gleicher Höhe.

Beispiel 10:
Es wird analog zu Beispiel 7 verfahren, wobei jedoch statt Ethanol 2% Wasser enthaltendes Aceton verwendet wird. Die Ausbeute ist mit der in Beispiel 7 erreichten vergleichbar.

Beispiel 11:
177,8 g racemische
cis-1,3-Dibenzyl-5-benzyloxycarbonyl-2-oxo-imidazolidine-4-carbonsäure
(erhältlich aus
cis-1,3-Dibenzylhexahydro-1H-furo[3,4-d]-imidazol-2,4,6-trion

und Benzylalkohol analog zu Beispiel 1a) und 117,8 g (+)-Dehydroabietylamin werden in 800 ml Toluol gelöst und nach Zugabe von 40 ml Wasser der Kristallisation bei 0° überlassen.
Ausbeute an
(4R, 5S)-cis-1,3-Dibenzyl-5-benzyloxycarbonyl-2-oxoimidazolidin-4-carbonsäure:
138,6 g (95% d.Th.); $[\alpha]_{365}^{25} = +45,2°$; optische Reinheit: 99%.
Die Mutterlauge wird zur Trockene eingeengt. Man nimmt den Rückstand in 600 ml Methyl-tert.-butylether auf und erhält nach Kristallisation bei Raumtemperatur 135,8 g (93% d.Th.)
(4S, 5R)-cis-1,3-Dibenzyl-5-benzyloxycarbonyl-2-oxoimidazolidin-4-carbonsäure;
$[\alpha]_{365}^{25} = +54,2°$; optische Reinheit: ≥99%.

Beispiel 12:
a) Die Suspension von 23,3 g
(4S, 5R)-cis-1,3-Dibenzyl-5-ethoxycarbonyl-2-oxo-imidazolidin-4-carbonsäure-(+)-dehydroabietylaminsalz

in 200 ml Wasser und 200 ml Essigester wird unter Rühren mit 35 ml 2N Schwefelsäure versetzt. Durch Filtration erhält man 12,18 g (90,6% d.Th.) (+)-Dehydroabietylaminhydrogensulfat. Im Filtrat wird die organische Phase abgetrennt und mit Wasser gewaschen. Nach Trocknen und Abziehen des Lösungsmittels sowie Kristallisation aus Diisopropylether verbleiben 12,7 g (94,8% d.Th.)
(4S, 5R)-cis-1,3-Dibenzyl-5-ethoxycarbonyl-2-oxo-imidazolidin-4-carbonsäure;
$[\alpha]_{365}^{25} = -23,8°$.

b) Die in Beispiel 12a erhaltenen 12,18 g (+)-Dehydroabietylaminhydrogensulfat werden in 200 ml Wasser angerührt und durch Zugabe von konz. Natronlauge auf pH 12 gebracht. Es wird zweimal mit je 100 ml Toluol extrahiert. Nach Waschen und Trocknen der vereinigten Extrakte dampft man ein und erhält so 8,9 g (88,9% d.Th.) zur erneuten Racematspaltung geeignetes (+)-Dehydroabietylamin.

Beispiel 13:
Analog zu Beispiel 12 werden erhalten:
(4S, 5R)-1,3-Dibenzyl-5-methoxycarbonyl-2-oxo-imidazolidin-4-carbonsäure:
$[\alpha]_{365}^{25} = -12{,}75°$.
(4R, 5S)-1,3-Dibenzyl-5-methoxycarbonyl-2-oxo-imidazolidin-4-carbonsäure:
$[\alpha]_{365}^{25} = +18{,}2°$.

Beispiel 14:
Die Reduktion der nach Beispiel 12 erhaltenen optisch aktiven
cis-1,3-Dibenzyl-5-ethoxycarbonyl-2-oxo-imid-azolidin-4-carbonsäuren
zu den entsprechenden optisch aktiven
1,3-Dibenzyl-tetrahydro-4H-furo[3,4-d]imidazol-2,4(1H)-dionen
mit Lithiumborhydrid wird analog zu dem von Gerecke et al., Helv. Chim. Acta 53 (1970) 991–999, beschriebenen Verfahren ausgeführt.
Ausbeute an
(3aS, 6aR)-1,3-Dibenzyl-tetrahydro-4H-furo[3,4-d]imidazol-2,4(1H)-dion
88,5% d.Th.; F: 119,4°; $[\alpha]_{365}^{25} = +204{,}6°$. Aus diesem Lacton wird gemäß DBP 2 058 234 oder DBP 2 331 244 D-(+)-Biotin erhalten.

Beispiel 15:
Analog zu Beispiel 14 werden die Enantiomeren
cis-1,3-Dibenzyl-5-methoxycarbonyl-2-oxo-imid-azolidin-5-carbonsäuren
mit Lithiumborhydrid reduziert. Dadurch erhält man:
(3aS, 6aR)-1,3-Dibenzyl-tetrahydro-4H-furo[3,4-d]-imidazol-2,4(1H)-dion;
97,4% d.Th.; $[\alpha]_{365}^{25} = +208°$ und
(3aR, 6aS)-1,3-Dibenzyl-tetrahydro-4H-furo[3,4-d]-imidazol-2,4(1H)-dion;
Ausbeute quantitativ: $[\alpha]_{365}^{25} = -206°$.

Beispiel 16:
Man legt 4 g Natriumborhydrid in 250 ml Tetrahydrofuran vor und erhitzt die Suspension zum Rückfluß. Innerhalb von 4 Stunden tropft man die Lösung von 60.8 g
(4S, 5R)-1,3-Dibenzyl-5-ethoxycarbonyl-2-oxo-imidazolin-4-carbonsäure-(+)-dehydroabietylaminsalz
(erhalten nach den Beispielen 1b, 2, 3 oder 4) in 150 ml Tetrahydrofuran zu der NaBH₄-Suspension und rührt anschließend 1 Std. unter weiterem Rückflußkochen nach.
Es wird auf 20° abgekühlt und durch tropfenweise Zugabe von 300 ml 3N Schwefelsäure zersetzt. Man zieht das Lösungsmittel unter vermindertem Druck weitgehend ab, saugt den anfallenden Niederschlag ab und wäscht mit Wasser nach.
Der Filterrückstand wird in Ethanol aufgenommen und durch Erwärmen gelöst. Beim Erkalten tritt Kristallisation ein. Nach Filtration und Nachwaschen mit Ethanol erhält man 27,3 g (93% d.Th.)
(3aS, 6aR)-1,3-Dibenzyltetrahydro-4H-furo[3,4-d]-imidazol-2,4(1H)-dion;
F 119°, $[\alpha]_{365}^{25} = +208{,}7°$ (c = 1, Benzol).
Aus der Mutterlauge erhält man nach Eindampfen und Behandlung mit Dichlormethan 33,1 g Dehydroabietylaminhydrogensulfat zurück.

Beispiel 17:
Zu 170 ml einer 0,15 molaren Diboran-Lösung in Tetrahydrofuran tropft man unter Rühren bei 0° langsam die Lösung von 33,4 g
(4R, 5S)-1,3-Dibenzyl-5-ethoxycarbonyl-2-oxo-imidazolidin-4-carbonsäure-(+)-dehydroabietylaminsalz
(hergestellt nach Beispiel 1b, 2, 4, 5 oder 6).
Nach beendeter Zugabe wird noch 5 Std. bei dieser Temperatur weitergerührt und anschließend analog Beispiel 16 mit verdünnter Schwefelsäure zersetzt und aufgearbeitet. Man erhält so 14,6 g (91% d.Th.)
(3aS, 6aR)-1,3-Dibenzyltetrahydro-4H-furo[3,4-d]-imidazol-2,4(1H)-dion;
F: 118,8°, $[\alpha]_{365}^{25}$ +206,4° (c = 1, Benzol).

Beispiel 18:
Analog Beispiel 16 wird das
d-1-Phenyl-2-p-tolylethanaminsalz der
(4S, 5R)-1,3-Dibenzyl-5-methoxycarbonyl-2-oxo-imidazolin-4-carbonsäure
(hergestellt nach EP-OS 92 194) in Tetrahydrofuran mit Natriumborhydrid reduziert. Dadurch erhält man
(3aS, 6aR)-1,3-Dibenzyltetrahydro-4H-furo[3,4-d]-imidazol-2,4(1H)-dion
in 89% d.Th.
Aus diesem Lacton wird gemäß DBP 2 058 234 oder 2 33 244 D-(+)-Biotin erhalten.

Beispiel 19:
Analog Beispiel 17 wird das
d-1-Phenyl-2-p-tolylethanaminsalz der
(4R, 5S)-1,3-Dibenzyl-5-methoxycarbonyl-2-oxo-imidazolidin-4-carbonsäure
(hergestellt nach EP-OS 92 194) in Tetrahydrofuran mit Diboran reduziert. Dadurch erhält man
(3aS, 6aR)-1,3-Dibenzyl-tetrahydro-4H-furo-[3,4-d]imidazol-2,4(1H)-dion
in 92% d.Th.

**Patentansprüche**

1. (+)-Dehydroabietylaminsalze von (4S, 5R)-und
(4R, 5S)-cis-1,3-Dibenzyl-5-alkyloxycarbonyl-2-oxoimidazolidin-4-carbonsäure,
worin -alkyl- Alkyl mit 1 bis 6 C-Atomen, Alkoxyalkyl mit 2 bis 8 C-Atomen, Cycloalkyl mit 3 bis 5 C-Atomen, Alkenyl mit 2 bis 6 C-Atomen, Benzyl oder 1- oder 2-Phenylethyl bedeutet.

2. (+)-Dehydroabietylaminsalze von (4S, 5R)- und
(4R, 5S)-cis-1,3-Dibenzyl-5-methoxycarbonyl-
   2-oxoimidazolidin-4-carbonsäure.

3. (+)-Dehydroabietylaminsalze von (4S, 5R)- und
(4R, 5S)-cis-1,3-Dibenzyl-5-ethoxycarbonyl-2-oxo-
   imidazolidin-4-carbonsäure.

4. Verfahren zur Herstellung von (3aS, 6aR)- und/oder
(3aR, 6aS)-1,3-Dibenzyl-hexahydro-1H-furo[3,4-d]-
   imidazol-2,4-dion aus
1,3-Dibenzyl-5-alkoxycarbonyl-2-oxoimidazo-
   lidin-4-carbonsäuren,
dadurch gekennzeichnet, daß man ein diastereomeres Salz aus einem optisch aktiven Amin und
einer optisch aktiven
1,3-Dibenzyl-5-alkoxycarbonyl-2-oxoimidazol-4-
   carbonsäure
mit komplexen Hydriden reduziert.

5. Verfahren nach Anspruch 4, dadurch gekennzeichnet, daß man das distereomere Halbestersalz an der Carboxylgruppe reduziert.

6. Verfahren nach Anspruch 4, dadurch gekennzeichnet, daß man das diastereomere Halbestersalz an der Alkoxycarbonylgruppe reduziert.

7. Verfahren nach einem der Ansprüche 4 bis 6,
dadurch gekennzeichnet, daß das optisch aktive
Amin (+)-Dehydroabietylamin ist.

8. Verfahren zur Herstellung von
(3aS, 6aR)-1,3-Dibenzylhexahydro-1H-furo[3,4-d]-
   imidazol-2,4-dion
nach einem der Ansprüche 4 bis 7, dadurch gekennzeichnet, daß man das (+)-Dehydroabietylaminsalz von (4S, 5R)-1,3-Dibenzyl-5-methoxy-
und/oder -5-ethoxy- und/oder
-5-benzyloxycarbonyl-2-oxo-imidazolidin-4-
   carbonsäure
mit Natriumborhydrid reduziert.

9. Verfahren zur Herstellung von
(3aS, 6aR)-1,3-Dibenzylhexahydro-1H-furo[3,4-d]-
   imidazol-2,4-dion
nach einem der Ansprüche 4 bis 7, dadurch gekennzeichnet, daß man das
(+)-Dehydroabietylaminsalz von (4R, 5S)-1,3-
   Dibenzyl-5-methoxy- und/oder
   -5-ethoxy- und/oder -5-benzyloxycarbonyl-
   2-oxoimidazolidin-4-carbonsäure
mit Diboran reduziert.

10. Verwendung eines nach den Ansprüchen 4
bis 9 erhaltenen optisch aktiven
1,3-Dibenzyl-hexahydro-1H-furo[3,4-d]imidazol-
   2,4-dions
zur Herstellung von D-(+)-Biotin.

## Claims

1. (+)-Dehydroabietylamine salts of (4S, 5R)- and
(4R, 5S)-cis-1,3-dibenzyl-5-alkyloxycarbonyl-
   2-oxo-imidazolidine-4-carboxylic acid,
in which -alkyl- is alkyl having 1 to 6 C atoms,
alkoxyalkyl having 2 to 8 C atoms, cycloalkyl
having 3 to 5 C atoms, alkenyl having 2 to 6 C
atoms, benzyl or 1- or 2-phenylethyl.

2. (+)-Dehydroabietylamine salts of (4S, 5R)- and
(4R, 5S)-cis-1,3-dibenzyl-5-methoxycarbonyl-
   2-oxoimidazolidine-4-carboxylic acid.

3. (+)-Dehydroabietylamine salts of (4S, 5R)- and
(4R, 5S)-cis-1,3-dibenzyl-5-ethoxycarbonyl-2-oxo-
   imidazolidine-4-carboxylic acid.

4. Process for preparing (3aS, 6aR)- and/or
(3aR, 6aS)-1,3-dibenzyl-hexahydro-1H-furo[3,4-d]-
   imidazole-2,4-dione
from 1,3-dibenzyl-5-alkoxycarbonyl-2-oxo-
   imidazolidine-4-carboxylic acids, characterized
in that a diastereomeric salt formed between an
optically active amine and an optically active
1,3-dibenzyl-5-alkoxycarbonyl-2-oxo-imidazole-
   4-carboxylic
acid is reduced with complex hydrides.

5. Process according to Claim 4, characterized
in that the diastereomeric half-ester salt is reduced at the carboxyl group.

6. Process according to Claim 4, characterized
in that the diastereomeric half-ester salt is reduced at the alkoxycarbonyl group.

7. Process according to any one of Claims 4 to
6, characterized in that the optically active amine
is (+)-dehydroabietylamine.

8. Process for preparing
(3aS, 6aR)-1,3-dibenzyl-hexahydro-1H-furo[3,4-d]-
   imidazole-2,4-dione
according to any one of Claims 4 to 7, characterized in that the (+)-dehydroabietylamine salt of
(4S, 5R)-1,3-dibenzyl-5-methoxy- and/or -5-eth-
oxy- and/or
-5-benzyloxycarbonyl-2-oxo-imidazolidine-4-
   carboxylic acid
is reduced with sodium borohydride.

9. Process for preparing
(3aS, 6aR)-1,3-dibenzyl-hexahydro-1H-furo[3,4-d]-
   imidazole-2,4-dione
according to any one of Claims 4 to 7, characterized in that the (+)-dehydroabietylamine salt of
(4R, 5S)-1,3-dibenzyl-5-methoxy- and/or
   -5-ethoxy- and/or -5-benzyloxycarbonyl-
   2-oxo-imidazolidine-4-carboxylic acid
is reduced with diborane.

10. Use of an optically active
1,3-dibenzyl-hexahydro-1H-furo[3,4-d]imidazole-
   2,4-dione
obtained according to Claims 4 to 9 for preparing
D-(+)-biotin.

## Revendications

1. Les sels de (+)-déshydroabiétylamine des
acides (4S, 5R)- et
(4R, 5S)-cis-1,3-dibenzyl-5-alkyloxy-carbonyl-2-
   oxo-imidazolidine-4-carboxyliques
dans lesquels la partie «-alkyl-» consiste en fait en
un groupe alkyle en C 1-C 6, alcoxyalkyle en C 2-C
8, cycloalkyle en C 3-C 5, alcényle en C 2-C 6,
benzyle ou 1- ou 2-phényléthyle.

2. Les sels de (+)-déshydroabiétylamine des
acides (4S, 5R)- et
(4R, 5S)-cis-1,3-dibenzyl-5-méthoxy-carbonyl-2-
   oxo-imidazolidine-4-carboxyliques.

3. Les sels de (+)-déshydroabiétylamine des acides (4S, 5R)- et (4R, 5S)-cis-1,3-dibenzyl-5-éthoxy-carbonyl-2-oxo-imidazolidine-4-carboxyliques.

4. Procédé de préparation de la (3aS, 6aR)- et/ou de la (3aR, 6aS)-1,3-dibenzyl-hexahydro-1H-furo[3,4-d]-imidazole-2,4-dione à partir d'acides 1,3-dibenzyl-5-alcoxycarbonyl-2-oxo-imidazoli-dine-4-carboxyliques, caractérisé en ce que l'on réduit à l'aide d'hydrures complexes un sel diastéréoisomère d'une amine possédant l'acitivité optique et d'un acide 1,3-dibenzyl-5-alcoxycarbonyl-2-oxo-imidazole-4-carboxylique possédant l'activité optique.

5. Procédé selon la revendication 4, caractérisé en ce que l'on réduit le sel d'hémiester diastéréoisomère sur le groupe carboxyle.

6. Procédé selon la revendication 4, caractérisé en ce que l'on réduit le sel d'hémiester diastéréoisomère sur le groupe alcoxycarbonyle.

7. Procédé selon l'une des revendications 4 à 6, caractérisé en ce que l'amine possédant l'activité optique est la (+)-déshydroabiéthylamine.

8. Procédé de préparation de la (3aS, 6aR)-1,3-dibenzyl-hexahydro-1H-furo[3,4-d]imidazole-2,4-dione selon l'une des revendications 4 à 7, caractérisé en ce que l'on réduit par le borohydrure de sodium le sel de (+)-déshydroabiétylamine de l'acide (4S, 5R)-1,3-dibenzyl-5-méthoxy- et/ou -5-éthoxy-et/ou -5-benzyloxycarbonyl-2-oxo-imidazolidine-4-carboxylique.

9. Procédé de préparation de la (3aS, 6aR)-1,3-dibenzyl-hexahydro-1H-furo[3,4-d]-imidazole-2,4-dione selon l'une des revendications 4 à 7, caractérisé en ce que l'on réduit par le diborane le sel de (+)-déshydroabiétylamine de l'acide (4R, 5S)-1,3-dibenzyl-5-méthoxy- et/ou -5-éthoxy- et/ou -5-benzyloxycarbonyl-2-oxo-imidazolidine-4-carboxylique.

10. Utilisation d'une 1,3-dibenzyl-hexahydro-1H-furo[3,4-d]imidazole-2,4-dione possédant l'activité optique, obtenue selon les revendications 4 à 9, pour la préparation de la D-(+)-biotine.